# EUROPEAN PATENT APPLICATION

(11) **EP 4 151 174 A1**
(43) Date of publication of application: **22.03.2023**
(21) Application number: 21315159.0
(22) Date of filing: 15.09.2021
(51) Int. Cl.: A61B 90/50, A61M 25/09, A61B 34/00, A61B 17/00

(54) **DEVICE FOR INSERTING AND EXCHANGING A SURGICAL INSTRUMENT**

(71) Applicant: Caranx Medical SAS, 75003 Paris (FR)
(72) Inventor: SEJOR, Eric, 06000 Nice (FR); SMITS, Jonas Victor Harmen, 3360 Bierbeek (BE); BERTHET-RAYNE , Pierre, 06800 Cagnes-sur-Mer (FR)
(74) Representative: Hepp Wenger Ryffel AG

(57) **Abstract**

The invention pertains to a device (50) for inserting and exchanging a surgical instrument (20) during percutaneous interventions, the device (50) comprising an axial displacement unit (4) designed and arranged to insert and retract a surgical instrument (20) along an insertion path (71) at an access site (70); a storage unit (21) having at least two cavities (A, B, C) designed and arranged to store at least one surgical instrument (20); and a positioning unit for positioning a surgical instrument (20) stored in the storage unit (21) with respect to the inserting unit (4) and the insertion path (71).

## Description

The present application relates to a device for inserting and exchanging a surgical instrument during percutaneous interventions:
A related device is disclosed in US 2020/0282186 A1. The apparatus includes a hemostasis valve, a base having a clamp releasably coupling a catheter to the base, a base drive member moving the base relative to the hemostasis valve along a first path, and a mechanism maintaining the position of an elongated medical device relative to the hemostasis valve while the catheter is being moved along the first path. However, among several disadvantages, this apparatus does not allow an automation or a remote operation.

It is an object of the present invention to provide an improved device which allows to gain access to a treatment site including a vascular anatomy, in particular during percutaneous surgical interventions in a remote-operated or automated manner. More particularly, it should allow performing, fully or partially, the insertion and exchange of surgical instruments into a patient anatomy without the presence of a sterile operator at the intervention site, in particular into an artery or vein during percutaneous surgical interventions.

According to the invention, the device for inserting and exchanging a surgical instrument comprises
- an axial displacement unit designed and arranged to insert and retract a surgical instrument along an insertion path at an access site,
- a storage unit having at least two cavities designed and arranged to store at least one surgical instrument,
- a positioning unit for positioning a surgical instrument stored in the storage unit with respect to the axial displacement unit and the insertion path.

The device may be used for minimally invasive surgery, for example for percutaneous interventions, endoscopy or laparoscopy. The instrument may be introduced through the access site and then to a treatment site within a patient's body. The treatment site may comprise an anatomy, in particular a vascular anatomy such as a vein or an artery, having a tubular shape or an organ. The treatment site may be remote from the access site. For example, in heart valve repair, the access site may be located at the top of a thigh whereas the treatment site is located in the area of a heart.

Preferably, at least one of the cavities may be designed to remain open during use and may allow the transfer of an instrument, e. g. a passage or a guide wire. The positioning unit may be a mobile robotic arm or an arm attached to a table or a fixed arm or any type of local robotic actuated fixture or other types of actuators.

In accordance with the invention, the positioning unit allows to selectively position one of two or more surgical instruments in such a way that the selected instrument can be moved to the access site by the axial displacement unit.

The device may contain a support with an interface with an opening to which a proximal inlet end of a dilator is attached or attachable. A distal outlet end of the at least two cavities may be alignable with the interface of the support. The dilator may be used to keep open the passage to the access site during exchange of an instrument. Alternatively, the device may be used. to pass an instrument to a treatment site which may be remote from the access site.

In a preferred embodiment, the storage unit may contain a barrel which is rotatable about a rotation axis by the positioning unit and which contains the at least two hollow cavities. Preferably, the cavities are cylinders having a circular cross-section. A rotatable barrel provides a space-saving arrangement of the cavities.

Alternatively, the storage unit may contain a tray which is translatable by the positioning unit, preferably along a linear translation axis, and which contains the at least two hollow cavities, preferably cylinders having a circular cross-section. The translation may also be along a non-linear path, for example a path defined by cam followers, belts, chains etc.

The storage unit may be fixed with respect to the support and may include a plurality of channels forming the cavities, wherein each of the channels has an individual proximal inlet and the channels converge to a joint distal outlet end. In this case the positioning unit may be designed to select an instrument in one of the channels and move it towards and out of the joint distal outlet end.

The device may further contain a guide wire which is placed upstream of the storage unit and insertable in a cavity of the storage unit or integrated in a cavity of the storage unit.

Within the present invention, a cavity is to be understood as an area adapted to receive an instrument. It does not necessarily have to be a separate closed space.

The guide wire may be held in a wire holder with a distal dispensing opening, and a proximal inlet end of one of the cavities may be alignable with the distal dispensing opening.

Furthermore, the device may contain an introducer and a needle, preferably a beveled hollow needle, which is located or adapted to be located in a lumen of the introducer.

Also with preference, the introducer and/or the needle may be axially movably arranged so as to be movable between the access site and one of the at least two cavities when a distal outlet end of the cavity and the interface of the support are aligned. The introducer and the needle may be both movable together or independently with respect to the barrel.

The device may further contain a locking arrangement comprising one or more locking elements for locking proximal inlet ends of the introducer and/or the needle at a distal outlet end of one of the cavities. This facilitates operation of the device.

The device may further comprise at least one spring, preferably two springs, at least one of the introducer and the needle compressing a respective spring in such way that when the locking arrangement is released, the introducer and the needle retract jointly or separately at least partially into the cavity. Alternatively, this actuation may be achieved by a linear motor, hydraulically, pneumatically or be a lever or gear mechanism.

Again with preference, in the at least two cavities, an instrument may be stored which is selected from the group of needle, dilator, guide wire, catheter, valve, valve delivery device, stent, stent delivery device, balloon, robotic device, micro robot, cauterization device, any type of flexible instruments such as graspers, endovascular nets such as filters, hemostatic (cauterization) and closure device.

The cavities may or may not be fully enclosed circumferentially. When they are not, an instrument may be laterally inserted into the cavity from the side.

The invention also pertains to a method of manipulating, in particular inserting or exchanging a surgical instrument, preferably with a device according to the invention. The method contains one or more of the following steps:
a) inserting a first surgical instrument stored in a storage unit along an insertion path at an access site by an axial displacement unit;
b) retracting the first surgical instrument from the access site along the insertion path,
d) positioning a second surgical instrument stored in the storage unit with respect to the axial displacement unit and the insertion path,
c) inserting the second surgical instrument along the insertion path at the access site by the axial displacement unit.

The invention will now be further described with reference to the attached drawings, in which
- Figure 1: shows a schematic drawing of a first embodiment of a device according to the invention;
- Figure 2: shows a detail of the first embodiment;
- Figure 3: shows a first method step of a first method of the invention;
- Figure 4: shows a second method step of the first method of the invention;
- Figure 5: shows a first step of a second method according to the invention;
- Figure 6: shows a second step of the second method according to the invention
- Figure 7: shows a third step of the second method according to the invention;
- Figure 8: shows a schematic drawing of a second embodiment of a device according to the invention;
- Figure 9: shows a schematic drawing of a third embodiment;
- Figure 10: shows a schematic drawing of a fourth embodiment.

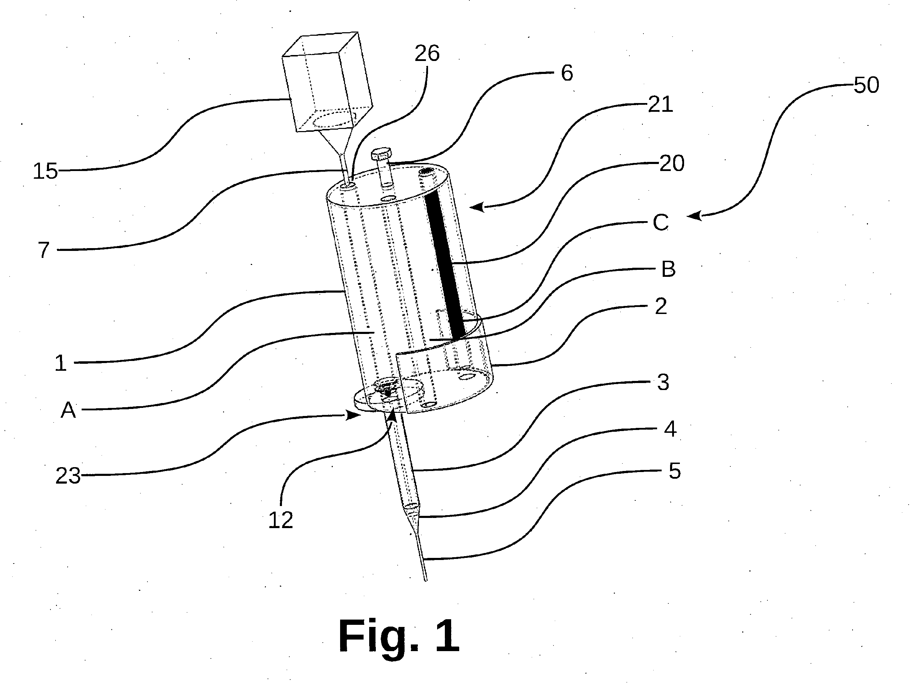

The device 50 shown in Figures 1 to 7 comprises a support 2 and a storage unit 21 having a barrel 1. The barrel 1 is rotatable with respect to the support 2 about a rotation axis 6. Rotation may be induced by a positioning unit. This may be achieved by techniques and mechanism that are known as such to the person skilled in the art, for example by a gear mechanism or a frictional coupling, which are not shown in the drawings.

Inside the barrel 1, three cylindrical cavities A, B, C are arranged, each of which extends parallel to the axis 6 and may store or temporarily receive at least one surgical instrument 20. The support 2 comprises an interface 23 with an opening to which a dilator 3 is attached at a proximal inlet end 11. By rotation of the barrel 1, a distal outlet end 12 of the cavities A, B, C is selectively alignable with the interface 23.

The device 50 further contains an intruder 4 having a lumen 17 in which a beveled hollow needle 5 is located. The introducer 4 and the needle 5 are axially movably arranged with respect to the barrel 1 and also with respect to each other so as to be movable between the access site 70 and one of the cavities A, B, C when a distal outlet end 12 of the cavity A, B, C and the interface 23 of the support 2 are aligned.

The device 50 further comprises a wire holder 15 with a distal dispensing opening 16 from which a guide wire 7 extends. Proximal inlet ends 26 of cavities A, B, C are selectively alignable with the distal dispensing opening 16 by rotation of the barrel 1. In this way, the guide wire 7 can be placed upstream of the storage unit 21 and insertable selectively in one of the cavities A, B, C of the barrel 1.

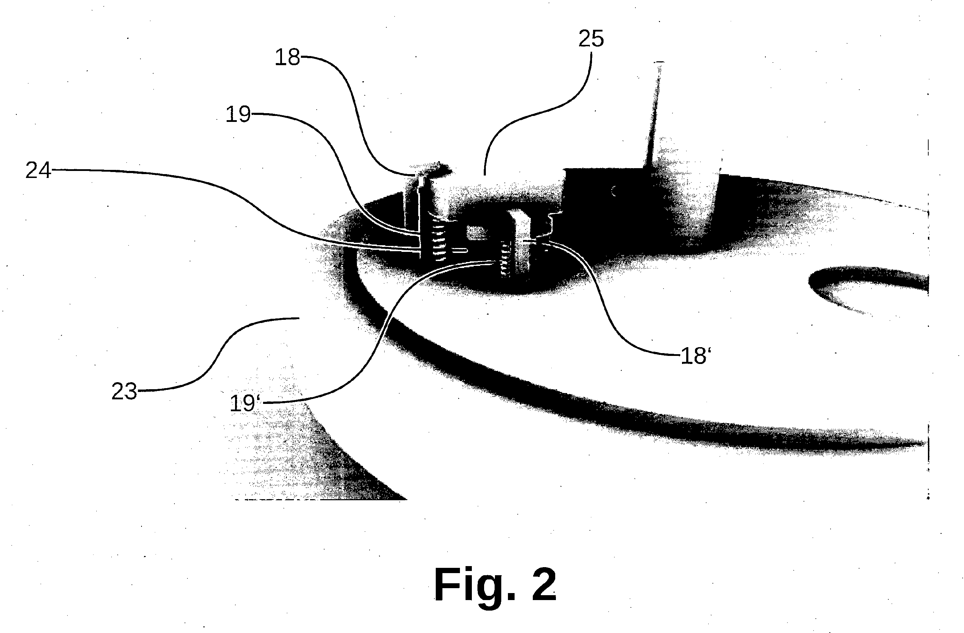

As shown in Figure 2, the device 50 further has a locking arrangement comprising two locking elements 18, 18' for locking a proximal inlet end 24 of the introducer 4 and a proximal end 25 of the needle 5 at a,distal outlet end 12 of one of the cavities A, B, C, respectively. Both the introducer 4 and the needle 5 compress a respective spring 19, 19' in such a way that when the locking elements 18, 18' are brought to a release position (for example by radial displacement or rotation), the introducer 4 and the needle 5 can retract jointly or separately at least partially into the cavity A.

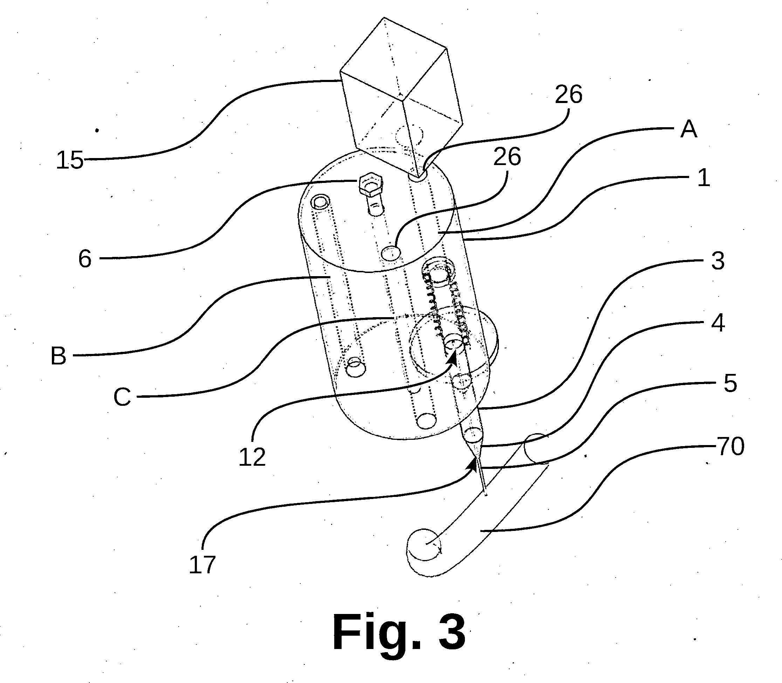

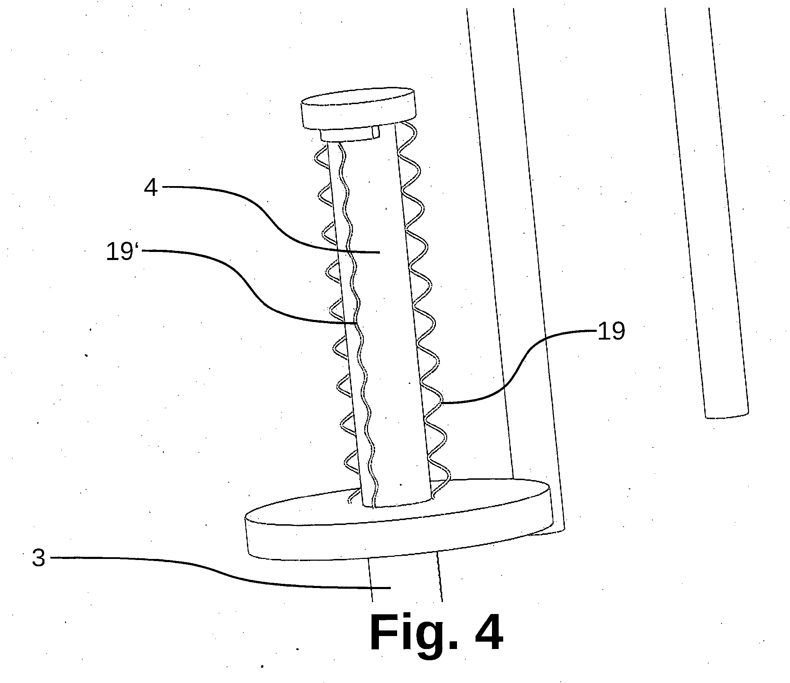

Figure 3 shows a position in which the introducer 4 and the needle 5 protrude from a distal end of the dilator 3. The position after retraction is shown in Figure 4 in which the introducer 4 and the needle 5 and the introducer 4 are reassembled in cavi-, ty A.

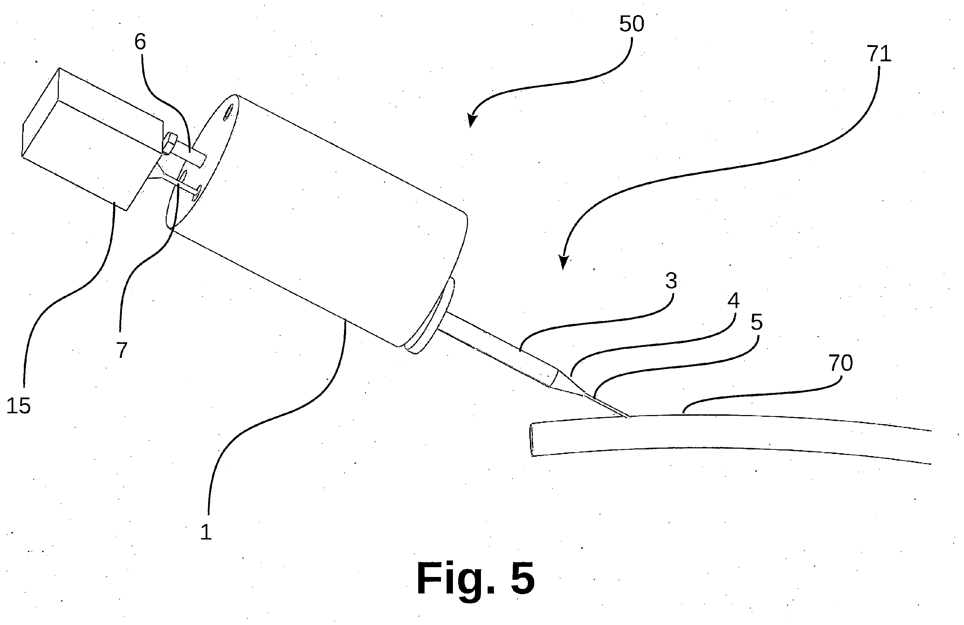

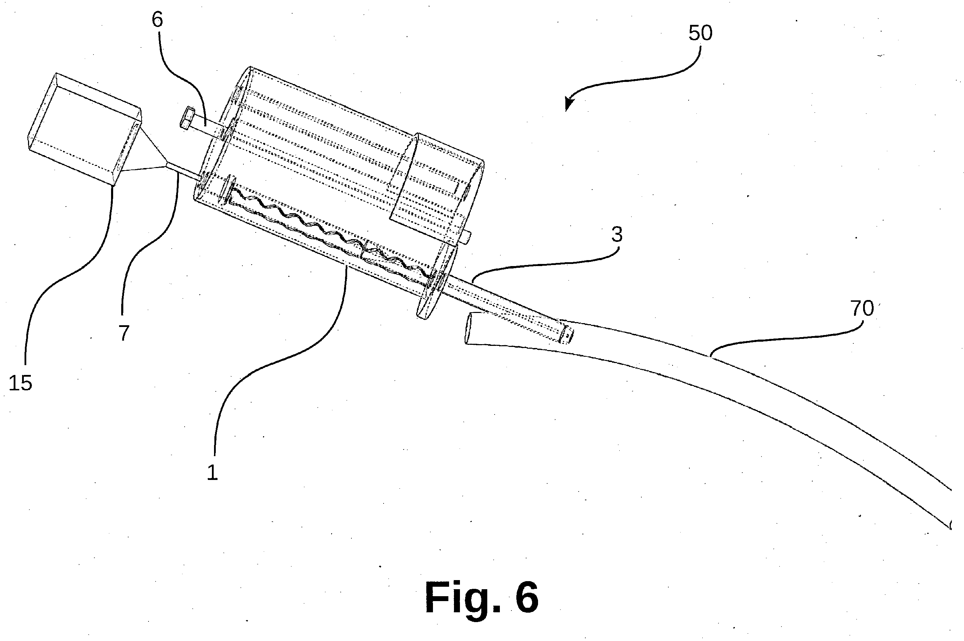

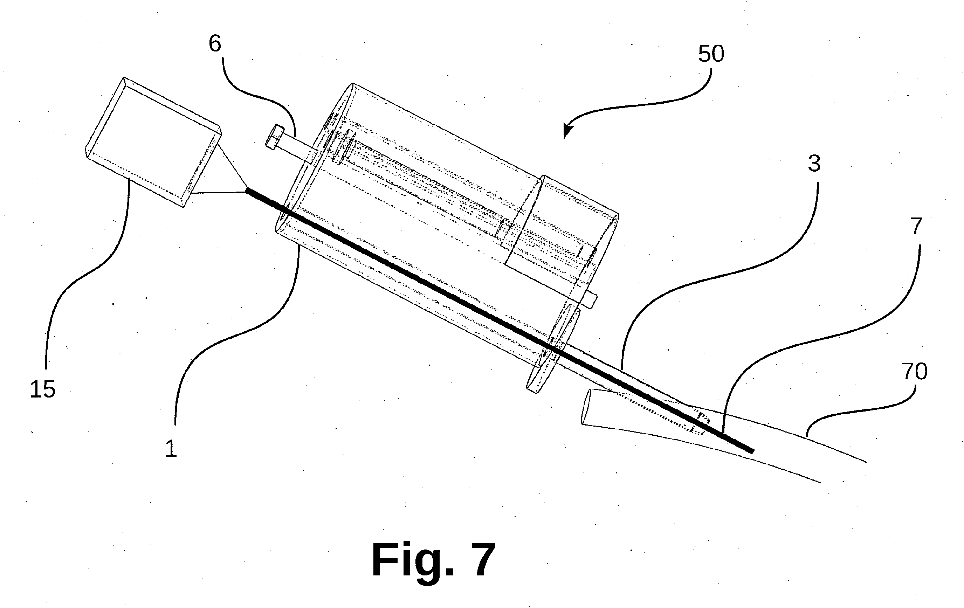

The device 50 may insert and retract a surgical instrument 20 along an insertion path 71 into a vessel 70, i. e. an access site, as illustrated in Figures 5 to 7: Figure 5 shows how the dilator 3 of the device 50 is inserted into a vessel 70 along an insertion path 71 until the dilator 3 has entered the vessel 70. As shown in Figure 6, the needle 5 and the introducer 4 are retracted. If the cavity A with the dilator 3 and the needle are aligned with the interface 23, the dilator 3 or needle 5 may be inserted. Subsequently, as depicted in Figure 7, the barrel 1 is rotated about rotation axis 6 and guide wire 7 is inserted through the barrel 1 and then into the vessel 70.

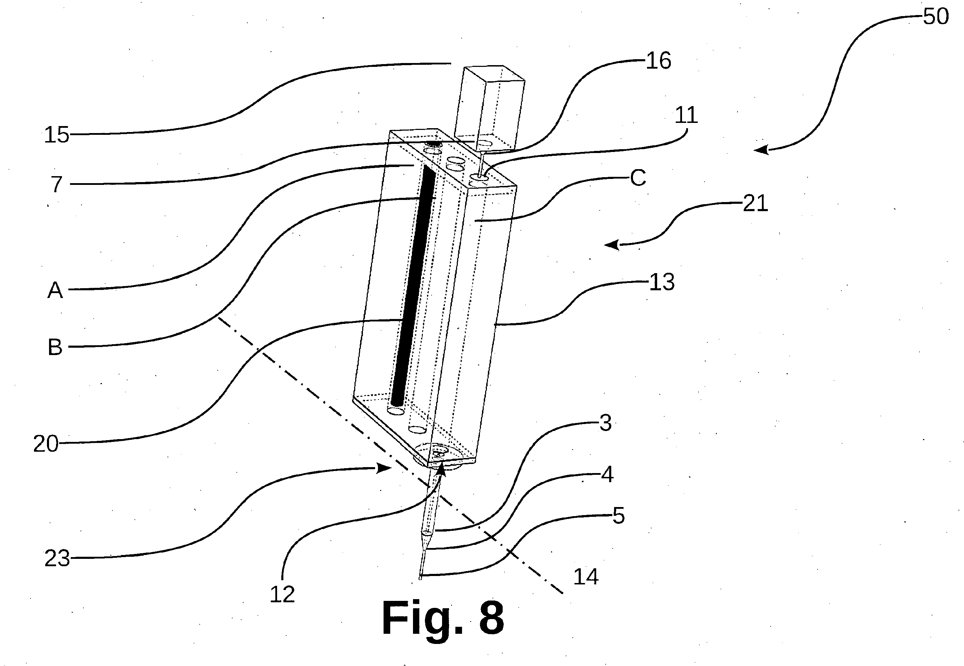

In the second embodiment shown in Figure 8, the storage unit 21 contains a tray 13 which is translatable along a linear translation axis 14. It contains three hollow cylinders A, B, C which each have a circular cross-section.

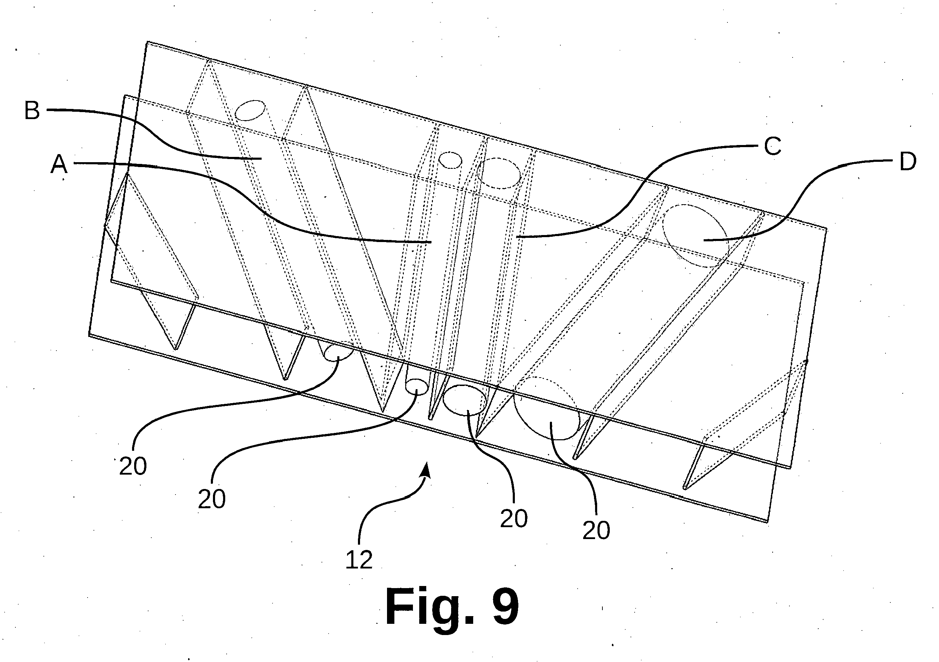

In a further embodiment shown in Figure 9, the storage unit 21 is fixed and includes a plurality of channels A, B, C and D which form the cavities. Each of the channels A, B, C and D has an individual proximal inlet end 11. The channels A, B, C and D converge to a joint distal outlet end 12. In each of the channels A, B, C, an instrument 20 is stored and dispensed through the joint distal outlet end 12.

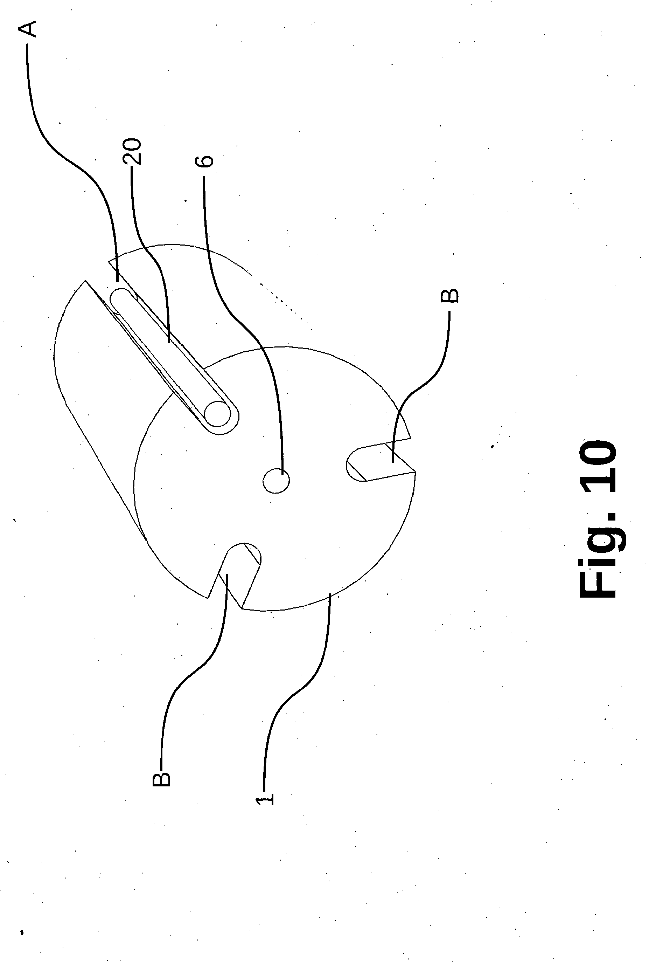

As shown in the embodiment shown in Figure 10, the cavities A, B and C do not need to be fully enclosed circumferentially. This allows to insert instruments 20 (of which only one is shown) laterally from the side.

## Claims

1. Device (50) for inserting and exchanging a surgical instrument (20), the device (50) comprising
- an axial displacement unit designed and arranged to insert and retract a surgical instrument (20) along an insertion path (71) at an access site (70),
- a storage unit (21) having at least two cavities (A, B, C, D) designed and arranged to store at least one surgical instrument (20),
- a positioning unit for positioning a surgical instrument (20) stored in the storage unit (21) with respect to the axial displacement unit and the insertion path (71).

2. The device (50) as claimed in claim 1,
wherein the device (50) contains a support (2) with an interface (23) to which a proximal inlet end (11) of a dilator (3) is attached or attachable , wherein a distal outlet end (12) of the at least two cavities (A, B, C, D) is selectively alignable with the interface (23) of the support (2).

3. The device (50) as claimed in one of the preceding claims, wherein the storage unit (21) contains a barrel (1) which is rotatable about a rotation axis (6) by the positioning unit, wherein the barrel (1) contains the at least two hollow cavities (A, B, C), preferably cylinders (A, B, C) having a circular cross-section.

4. The device (50) as claimed in one of claims 1 and 2,
wherein the storage unit (21) contains a tray (13) which is translatable by the positioning unit, preferably along a linear translation axis (14) and which contains the at least two hollow cavities, preferably cylinders (A, B, C) having a circular cross-section.

5. The device (50) as claimed in one of claims 1 and 2,
wherein the storage unit (21) is fixed with respect to the support (2) and includes a plurality of channels (A, B, C, D) forming the cavities, wherein each of the channels (A, B, C, D) has an individual proximal channel inlet end and the channels (A, B, C, D) converge to.a joint distal outlet end (12).

6. The device (50) as claimed in one of the preceding claims, wherein the device (50) contains a guide wire (7) which is placed upstream of the storage unit (21) and insertable in a cavity of the storage unit (21) or integrated in a cavity (A, B, C, D) of the storage unit (21).

7. The device (50) as claimed in claim 6,
wherein the guide wire (7) is held in a wire holder (15) with a distal dispensing opening (16) and wherein a proximal inlet end (26) of a cavity (A, B, C, D) is selectively alignable with the distal dispensing opening (16).

8. The device (50) as claimed in one of the preceding claims, wherein the device (50) contains an introducer (4) and a needle (5), preferably a beveled hollow needle, located in a lumen (17) of the introducer (4).

9. The device (50) as claimed in claim 8,
wherein the introducer (4) and/or the needle (5) are axially movably arranged so as to be movable between the access site (70) and one of the at least two cavities (A, B, C, D) when a distal outlet end (12) of the cavity (A, B, C, D) and the interface (23) of the support (2) are aligned, and the device (50) further has a locking arrangement comprising one or more locking elements (18, 18') for locking a proximal end (24) of the introducer (4) and a proximal end (25) of the needle (5) at a distal outlet end (12) of one of the cavities (A, B, C).

10. The device (50) as claimed in claim 9,
the device (50) further comprising at least one spring (19, 19'), preferably two springs (19, 19'), at least one of the introducer (4) and the needle (5) compressing a respective spring (19, 19') in such a way that when the locking arrangement (18, 18') is released, the introducer (4) and the needle (5) retract jointly or separately at least partially into the cavity (A, B, C, D).

11. The device (50) as claimed in one of the preceding claims, wherein in the at least two cavities (A, B, C, D), an instrument (20) is stored which is selected from the group of needle (5), dilator (3), guide wire (7), catheter, valve, valve delivery device, stent, stent delivery device, balloon, robotic device, micro robot, cauterization device, any type of flexible instruments such as graspers, endovascular nets such as filters, hemostatic (cauterization) and closure device.
